# NEW EUROPEAN PATENT SPECIFICATION

(11) **EP 2 272 476 B2**
(45) Date of publication and mention of the opposition decision: **04.01.2017**
(45) Mention of the grant of the patent: 22.01.2014
(21) Application number: 09738668.4
(22) Date of filing: 23.03.2009
(51) Int. Cl.: A61F 13/42, A61F 13/49

(54) **Absorbent Article**
SAUGFÄHIGER ARTIKEL
ARTICLE ABSORBANT

(30) Priority: 01.05.2008 JP 2008119993
(43) Date of publication of application: 12.01.2011
(73) Proprietor: Unicharm Corporation, Ehime 799-0111 (JP)
(72) Inventor: KAWAKAMI, Yusuke, Kanonji-shi Kagawa 769-1602 (JP); KASHIWAGI, Mari, Kanonji-shi Kagawa 769-1602 (JP)
(74) Representative: Dolleymores
(86) International application number: PCT/JP2009/055579
(87) International publication number: WO 2009/133731

(56) References cited:
- EP-A1- 0 021 492
- FR-A1- 2 733 146
- JP- - 11 004 852
- JP- - 59 199 801
- JP- - 2007 236 865
- JP- - 2007 252 659
- JP-A- 11 004 852
- JP-A- 59 199 801
- JP-A- 2007 175 390
- JP-A- 2007 175 390
- JP-A- 2007 236 865
- JP-A- 2007 252 659
- JP-A- 2008 099 947
- US- - 4 738 674
- US- - 6 527 756
- US- - 20040 158 212
- US-A- 4 192 311
- US-A- 4 507 121
- US-A- 5 354 289

## Description

### TECHNICAL FIELD

The present invention relates to absorbent articles and more particularly to absorbent articles such as disposable diapers, toilet-training pants or incontinent briefs.

### RELATED ART

Disposable diapers including composition adapted to indicate whether urination has occurred or not is known, for example, from the disclosure of JP 2007-252659 A (PATENT DOCUMENT 1). According to the disclosure of PATENT DOCUMENT 1, the diaper has an inner sheet lying on the side facing the wearer's skin, an outer sheet lying on the side facing a wearer's garment and an absorbent structure sandwiched between these inner and outer sheets. The outer sheet is coated on its side facing the absorbent structure with a hot melt composition containing pH indicator adapted to undergo color change depending on the value of pH.

This diaper of known art further comprises a hydrophilic intermediate sheet between the hot melt composition and the absorbent structure. The hot melt composition might be partially solved in the moisture derived from urination. If the composition partially solved in the moisture is transferred to the absorbent structure, the color of the composition might be misidentified as the color of urine. Interposition of the intermediate sheet prevents the composition partially solved in the moisture from being transferred to the absorbent structure and thereby prevents the color of the composition being misidentified as the color of urine.

[PATENT DOCUMENT 1] JP 2007-252659 A

### DISCLOSURE OF THE INVENTION

### PROBLEM TO BE SOLVED BY THE INVENTION

However, the intermediate sheet employed in this known art is hydrophilic and therefore the moisture such as urine having been absorbed by the absorbent structure is sometimes transferred through the intermediate sheet to the hot melt composition. With such diaper of prior art, when the amount of water vapor within the diaper may increase because the wearer becomes sweaty or the diaper is put on the wearer's body immediately after his or her bath, sweat or water vapor may sometimes cause color change of the hot melt composition. Color change developed by the composition due to sweat or water vapor may cause helper or parent to misidentify as if urination would have occurred. Furthermore, such color change developing in the composition without occurrence of urination would result in another disadvantage that the urination really occurring can not be identified because the color change has already been developed before the real urination occurs.

It is an object of the present invention to provide an absorbent article enabling the presence of moisture, for example, derived from urination to be correctly recognized.

### MEASURE TO SOLVE THE PROBLEM

The object set forth above is achieved, according to the present invention, by an absorbant article as recited by claim 1.

### EFFECT OF THE INVENTION

According to the present invention, the moisture visualizing element is provided on the side thereof facing the wearer's skin with a hydrophobic cover sheet having a water pressure resistance in a range of 40 to 500 mm. In consequence, while a limited amount of the moisture such as sweat or water vapor is reliably prevented by the cover sheet from coming in contact with the moisture visualizing element, a large amount of the moisture due to real urination can come in contact with the moisture visualizing element and thereby prevent any misidentification due to sweat or water vapor.

The cover sheet has a sufficient area to cover the moisture visualizing element completely in the longitudinal direction as well as in the transverse direction of the moisture visualizing element, the cover sheet can completely overlap the moisture visualizing element to avoid the misidentification.

### BRIEF DESCRIPTION OF THE DRAWINGS

[FIG. 1] Plan view of a diaper according to one embodiment of the present invention.
[FIG. 2] Sectional view taken along the line II-II in Fig. 1.
[FIG. 3] Exploded perspective view corresponding to Fig. 1.

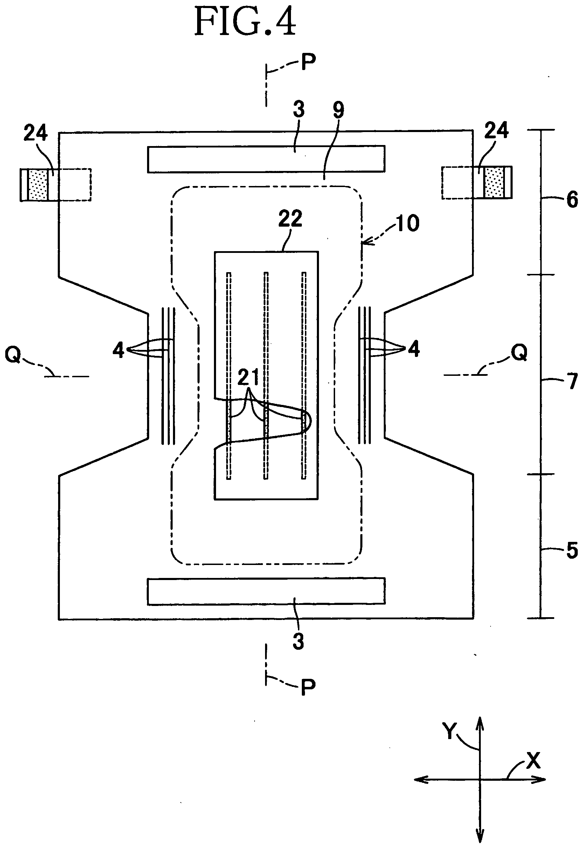

[FIG. 4] Partially cutaway schematic diagram corresponding to Fig. 1.

### IDENTIFICATION OF REFERENCE NUMERALS USED IN THE DRAWINGS

- 1: diaper
- 5: front waist region
- 6: rear waist region
- 7: crotch region
- 8: inner sheet
- 9: outer sheet
- 10: liquid-absorbent structure
- 21: moisture visualizing element
- 22: barrier sheet

### DETAILED DESCRIPTION OF THE PREFERRED EMBODIMENTS

Details of the present invention will be more fully understood from the following exemplary description given hereunder with respect to the disposable diaper as one embodiment of an absorbent article.

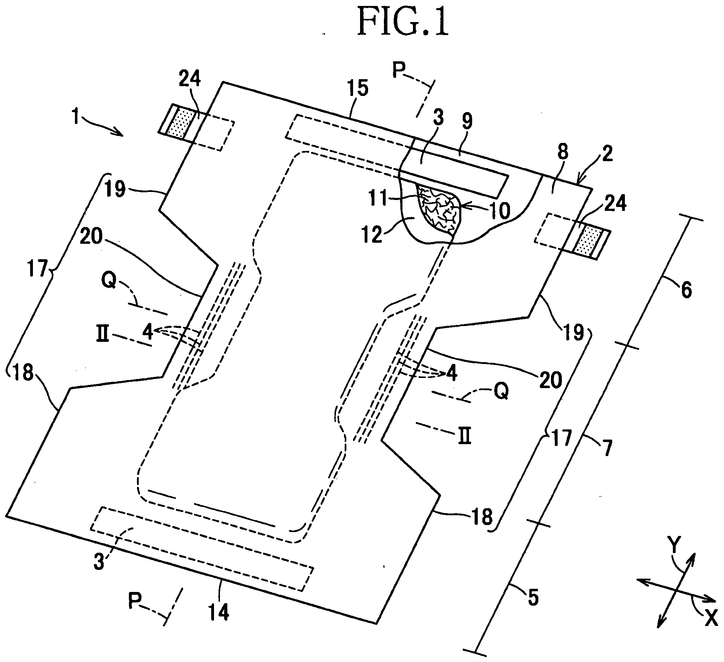

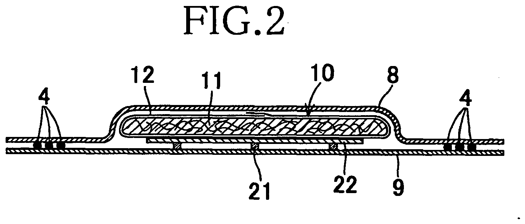

Figs. 1 through 3 illustrate a first embodiment of the invention wherein respective elastic members are shown as elasticity thereof being inactivated. Fig. 1 is a plan view showing a diaper 1 of so-called open-type flatly developed and partially cutaway for inconvenience of illustration and Fig. 2 is a sectional view taken along the line II-II in Fig. 1. As illustrated, the diaper 1 comprises a chassis 2, waist elastic members 3 and leg elastic members 4. The chassis 2 comprises a front waist region 5, a rear waist region 6 and a crotch region 7 extending between the front and rear waist regions 5, 6, these regions 5, 6 and 7 being contiguous one to another in a longitudinal direction Y. The chassis 2 includes a liquid-pervious inner sheet 8 defining an inner side facing the wearer's skin and a liquid-impervious outer sheet 9 defining an outer side facing the wearer's garment. The chassis 2 is provided between these inner and outer sheets 8, 9 with a liquid-absorbent structure 10 at least occupying the crotch region 7.

The liquid-absorbent structure 10 comprises a liquid-absorbent core 11 formed, for example, from fluff pulp and a diffusion sheet 12 such as tissue paper with which the liquid-absorbent core 11 is wrapped.

he chassis 2 has front and rear ends 14, 15 opposed to each other in the longitudinal direction Y and extending in a transverse direction X along which respective waist elastic members 3 are attached to the chassis 2. Each of these waist elastic members 3 is formed of an elasticized sheet attached to the chassis 2 under tension in the transverse direction X.

The chassis 2 has a pair of side edges 17 opposed to each other in the transverse direction X and extending in the longitudinal direction Y. Each of these side edges 17 comprises a front side edge 18 extending in the front waist region 5, a rear side edge 19 extending in the rear waist region 6 and a leg side edge 20 extending in the crotch region 7. The opposite side edges 17 describe curvatures along the respective leg side edges 20 which are convex toward a longitudinal center line P-P bisecting a dimension of the diaper 1 in the transverse direction X so that the chassis 2 as a whole has an concave-shape.

The opposite side edges 17 are provided with a plurality of leg elastic members 4 extending along the leg side edges 20. Specifically, these leg elastic members 4 are attached to the chassis 2 under tension so as to be sandwiched between the inner and outer sheets 8, 9. The leg elastic members 4 bias the leg side edges 20 to fit around the wearer's legs and thereby prevent body waste such as urine from leaking beyond the leg side edges 20.

The rear side edges 19 are provided on the side of the outer sheet 9 with fastener members 24 extending outward in the transverse direction X so that these fastener members 24 would be engaged with the outer sheet 9 in the front waist region 5 to put the diaper 1 on the wearer's body.

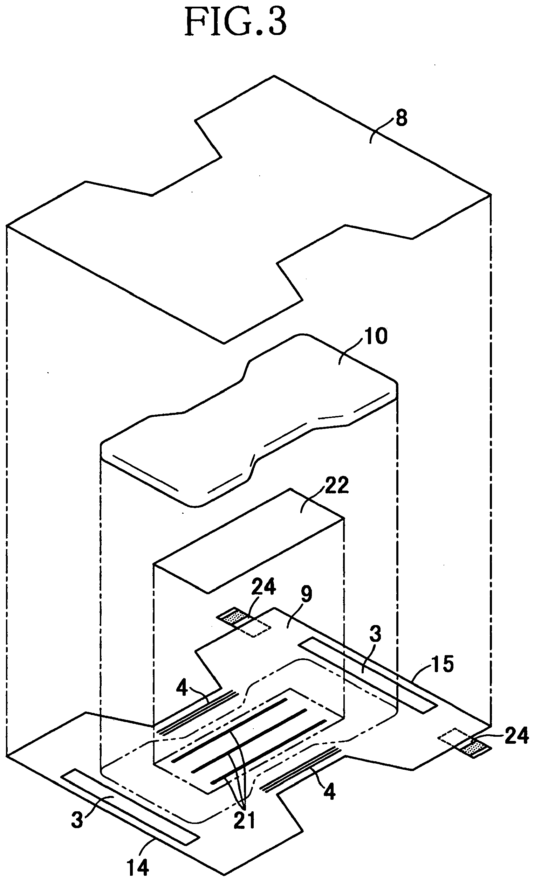

Fig. 3 is a plan view corresponding to Fig. 1 with the inner sheet 8 and the liquid-absorbent structure 10 eliminated therefrom. As will be apparent from Fig. 3, a moisture visualizing element 21 is provided on the side of the outer sheet 9 facing the wearer's skin, i.e., facing the liquid-absorbent structure 10. The moisture visualizing element 21 is formed by coating the outer sheet 9 with hot melt composition comprising pressure-sensitive adhesive ingredient mixed with pH indicator. The moisture visualizing element 21 formed in this manner develops a color change upon contact with moisture and thereby visualizes the presence of moisture through such change of color. Two or more stripes each forming the moisture visualizing element 21 extend in the longitudinal direction Y at least across the crotch region 7. In this first embodiment, these moisture visualizing elements 21 extend across the crotch region 7 further into the front and rear waist regions 5, 6.

A cover sheet 22 is attached between the moisture visualizing elements 21 and the liquid-absorbent structure 10. The cover sheet 22 has a sufficient area to cover all the moisture visualizing elements 21 completely in the longitudinal direction as well as in the transverse direction and bonded to at least one of the outer sheet 9 and the liquid-absorbent structure 10.

With the arrangement as has been described above, upon urination by the wearer, urine passes through the inner sheet 8 and is absorbed by the liquid-absorbent structure 10. Then a portion of urine absorbed by the liquid-absorbent structure 10 comes in contact with the moisture visualizing elements 21 via the cover sheet 22. Upon contact with urine, the moisture visualizing elements 21 develop change of color. The moisture visualizing elements 21 are adapted to be visible through the outer sheet 9 and, if the wearer is a baby, a helper or a parent of this baby can perceive that urination has occurred by observing such change of color.

As the cover sheet 22, hydrophobic fibrous nonwoven fabric exhibiting the water pressure resistance in a range of 40 to 500 mm is used. The water pressure resistance was measured in accordance with JIS-L1092. More specifically, after preparation of cover sheet test pieces each dimensioned to be 160 mm x 160 mm, a level vial filled with distilled water placed below the test piece was moved upward at a rate of 10 cm/min until a drop of water appeared on the sheet surface and a water level at this moment was obtained as the water pressure resistance.

TABLE 1 shows results of the water pressure resistance test having been conducted on Example 1, Example 2, Control 1 and Control 2. Example 1 was the diaper using the cover sheet comprising a hydrophobic SMS (spun bond/melt blow/spun bond) fibrous nonwoven fabric, Example 2 was the diaper using the cover sheet comprising a hydrophobic air-through fibrous nonwoven fabric. Control 1 was the diaper using the hydrophilic air-through fibrous nonwoven fabric and Control 2 was the diaper using no cover sheet.

The water pressure resistance test was conducted on the respective Examples as well as on the respective Controls to determine the water pressure resistance exhibited by each of the cover sheets in Examples and Controls. As for Control 2, no result of the water pressure resistance test was indicated in TABLE 1 because any kind of the cover sheet is not incorporate in Control 2. Also, TABLE 1 indicates the result of determination whether the moisture visualizing element used with various types of the cover sheet undergoes color change or not. The result of the test conducted on Control 2 indicates the result obtained in the case wherein the moisture visualizing element is not combined with any type of the cover sheets. In this test to determine whether the moisture visualizing element undergoes color change or not, in order to reproduce the situation in which the wearer becomes sweaty or the diaper is put on the wearer's body immediately after his or her bath, the diaper was put around a paper cylinder at a room temperature of 50°C and at a relative humidity of 80% and the determination whether the moisture visualizing element develops any change of color or not was made after 1 hour, 2 hours and 3 hours.

**[TABLE 1]**

| | Water pressure-resistance (mm) | Moisture visualizing element color change test | | |
|---|---|---|---|---|
| | | After 1 hr | After 2 hrs | After 3 hrs |
| Ex. 1 | 87.2 | No color change | No color change | No color change |
| Ex. 2 | 49.8 | No color change | No color change | No color change |
| Con. 1 | 11.8 | No color change | Slight color change | Color change |
| Con. 2 | - | Color change | Color change | Color change |

As indicated in TABLE 1, Example 1 exhibited a water pressure resistance of 87.2 mm, Example 2 exhibited a water pressure resistance of 49.8 mm and Control 1 exhibited a water pressure resistance of 11.8 mm. Control 2 contains no cover sheet combined therewith and therefore exhibited no result to be indicated in the associated column in TABLE 1. It should be appreciated that each of the water pressure resistance values indicated in TABLE 1 is the average value obtained from values measured 10 times.

On whether the moisture visualizing elements develop any change, in both Examples 1 and 2, the moisture visualizing elements develop no change one to three hours after the test had been started and it was demonstrated that the moisture visualizing elements in these two Examples are free from any change due to sweat or water vapor. Although the moisture visualizing elements in Control 1 also developed no change one hour after the test had been started, a slight change was observed two hours after and such change became fully significant three hours after the test had been started. In Control 2, the moisture visualizing elements developed a significant change already one hour after the test had been started. It is concluded from such test result that the moisture visualizing elements combined with the cover sheet exhibiting a water pressure resistance lower than 40 mm as those in Controls 1 and 2 develop a change due to sweat or water vapor. In contrast, the moisture visualizing elements combined with the cover sheets each exhibiting a water vapor resistance of 40 mm or higher as those in Examples 1 and 2 develop no change due to sweat or water vapor and minimize a possibility that such sweat or water vapor might cause a helper or a parent to misidentify the change due to sweat or water vapor as if urination has occurred.

To reproduce a condition of urination, 80 cc of water was poured onto the liquid-absorbent structure 10 of the diaper 1 and then a weight of 3.5 kg was placed on it for each of Example 1, Example 2, Control 1 and Control 2. Five minutes after, it was determined whether the moisture visualizing elements had developed any change or not. It was confirmed on the basis of this test that the moisture visualizing elements develop a change without exception and therefore develop in response to urination. More specifically, urination can be properly recognized without misidentifying sweat or water vapor as occurrence of urination so far as Examples 1 and 2 are concerned.

The water pressure resistance of the cover sheet 22 is set herein to the range of 40 to 500 mm. If the water pressure resistance is lower than 40 mm, the moisture visualizing elements might develop any change due to sweat or water vapor as in the case of Controls 1 and 2 and lead to misidentification as if urination has occurred. If the water pressure resistance of the cover sheet 22 is higher than 500 mm, the cover sheet 22 may become unacceptably thick or stiff and degrade feeling to wear the diaper. Also, the excessively high water pressure resistance might make it difficult for the moisture visualizing elements to develop a significant change when urination has occurred.

While an SMS fibrous nonwoven fabric or air-through fibrous nonwoven fabric is used as the cover sheet 22 in the illustrated embodiment of the invention, it is possible to use, for example, a spun lace nonwoven fabric or resin bond nonwoven fabric as the cover sheet so far as the water pressure resistance is in the range of 40 to 500 mm.

The cover sheet 22 preferably has a ventilation rate in a range of 0.01 to 0.15 KPa·S/m². With respect to the ventilation rate, the cover sheet 22 was tested five times for each test piece using a measuring instrument KES-F8 manufactured by KATO TECH CO., LTD. and five measured values for each test piece were averaged to obtain the ventilation rate of this test piece. If the ventilation rate is lower than 0.01 KPa·S/m², the cover sheet 22 may let sweat or water vapor pass through the cover sheet and cause the moisture visualizing element 21 to develop color change. If the ventilation rate is higher than 0.15 KPa·S/m², the interior of the diaper may become stuffy and cause the wearer to suffer from skin trouble such as rash.

It is possible without departing from the scope of the invention to provide the inner sheet 8 on its side facing the wearer's skin with leak-barrier cuffs. The leak-barrier cuffs may be of the conventional type so far as these leak-barrier cuffs are effective to prevent bodily fluids such as urine from leaking beyond the leg edges.

While this embodiment uses hot melt composition comprising pressure-sensitive adhesive ingredient and pH indicator mixed therewith as the moisture visualizing elements, the present invention is not limited to this and any other composition can be used as the moisture visualizing elements 21 so far as color development, color fade or color change occurs upon contact with moisture to suggest the presence of moisture. Instead of using the hot melt composition, it is also possible to print the moisture visualizing elements directly on the diaper 1 with appropriate ink or to interpose the moisture visualizing elements in the form of a separately prepared sheet. While the moisture visualizing elements 21 extend across the crotch region 7 in the longitudinal direction Y in the illustrated embodiment, it is possible to provide these moisture visualizing elements 21 so as to extend in the transverse direction X or it is also possible to provide these moisture visualizing elements 21 in the form of graphics printed with the hot melt composition or the like.

## Claims

1. An absorbent article having a longitudinal direction, a transverse direction, a side facing the wearer's skin, a side facing a wearer's garment, a front waist region, a rear waist region and a crotch region extending between said front and rear waist regions wherein said front and rear regions and said crotch region are contiguous one to another in said longitudinal direction, and comprising an inner sheet lying on said side facing wearer's skin, an outer sheet lying on said side facing the wearer's garment, a liquid-absorbent structure sandwiched between said inner and outer sheets and occupying at least said crotch region and moisture visualizing elements formed on a side of said outer sheet facing said liquid-absorbent structure and adapted to develop change of optical effect upon contact with moisture, said absorbent article being **characterized in that**:
**the liquid-absorbent structure comprises a liquid-absorbent core and a diffusion sheet with which the absorbent core is wrapped;**
said moisture visualizing element is provided on a side thereof facing the wearer's skin with a hydrophobic cover sheet having a water pressure resistance in a range of 40 to 500 mm;
and said cover sheet has a sufficient area to cover said moisture visualizing element completely in said longitudinal direction as well as in said transverse direction of said moisture visualizing element.

## Patentansprüche

1. Saugfähiger Artikel mit einer Längsrichtung, einer Querrichtung, einer der Haut des Trägers zugekehrten Seite, einer der Bekleidung des Trägers zugekehrten Seite, einem vorderen Taillenbereich, einem hinteren Taillenbereich und einem sich zwischen dem besagten vorderen und dem besagten hinteren Taillenbereich erstreckenden Schrittbereich, wobei der besagte vordere und der besagte hintere Taillenbereich und der besagte Schrittbereich in der besagten Längsrichtung aneinander angrenzen, und mit einer auf der besagten der Haut des Trägers zugekehrten Seite liegenden Innenlage, einer auf der besagten der Bekleidung des Trägers zugekehrten Seite liegenden Außenlage, einer Flüssigkeit aufnehmenden Struktur, die zwischen der besagten Innenlage und der besagten Außenlage liegt und mindestens den besagten Schrittbereich belegt, und mit Feuchtigkeitsvisualisierungselementen, die auf einer der besagten Flüssigkeit aufnehmenden Struktur zugekehrten Seite der besagten Außenlage ausgebildet sind und die bei Kontakt mit Feuchtigkeit zur Entwicklung einer Änderung der optischen Wirkung ausgelegt sind, **dadurch gekennzeichnet, dass**:
die Flüssigkeit aufnehmende Struktur einen Flüssigkeit aufnehmenden Kern und eine Diffusionslage, mit welcher der Flüssigkeit aufnehmende Kern umhüllt ist, umfasst;
das besagte Feuchtigkeitsvisualisierungselement auf einer der Haut des Trägers zugekehrten Seite eine Wasser abweisende Decklage aufweist, die einen Wasserdruckwiderstand im Bereich von 40 bis 500 mm hat;
und dass die besagte Decklage eine Fläche hat, die groß genug ist, um das besagte Feuchtigkeitsvisualisierungselement in der besagten Längsrichtung sowie in der besagten Querrichtung des besagten Feuchtigkeitsvisualisierungselements vollständig zu bedecken.

## Revendications

1. Article absorbant ayant une direction allant dans le sens longitudinal, une direction allant dans le sens transversal, un côté orienté vers la peau de l'utilisateur, un côté orienté vers le vêtement de l'utilisateur, une région avant au niveau de la taille, une région arrière au niveau de la taille et une région au niveau de l'entrejambe s'étendant entre lesdites régions avant et arrière au niveau de la taille dans lequel lesdites régions avant et arrière et ladite région au niveau de l'entrejambe sont contiguës les unes par rapport aux autres dans ladite direction allant dans le sens longitudinal, et comportant une feuille intérieure reposant sur ledit côté orienté vers la peau de l'utilisateur, une feuille extérieure reposant sur ledit côté orienté vers le vêtement de l'utilisateur, une structure absorbant les liquides prise en sandwich entre lesdites feuilles intérieure et extérieure et occupant au moins ladite région au niveau de l'entrejambe et des éléments de visualisation d'humidité formés sur un côté de ladite feuille extérieure orientés vers ladite structure absorbant les liquides et adaptés pour développer un changement d'effet optique dès le contact avec de l'humidité, ledit article absorbant étant **caractérisé en ce que** :
la structure absorbant les liquides comporte une partie centrale absorbant les liquides et une feuille de diffusion au moyen de laquelle la partie centrale absorbante est enveloppée ;
ledit élément de visualisation d'humidité est mis en oeuvre sur un côté de celui-ci orienté vers la peau de l'utilisateur avec une feuille de couverture hydrophobe ayant une résistance à la pression d'eau de l'ordre de 40 à 500 mm ;
et ladite feuille de couverture a une surface suffisante pour couvrir ledit élément de visualisation d'humidité entièrement dans ladite direction allant dans le sens longitudinal ainsi que dans ladite direction allant dans le sens transversal dudit élément de visualisation d'humidité.
